# EUROPÄISCHE PATENTSCHRIFT

(11) **EP 1 470 184 B1**
(45) Veröffentlichungstag und Bekanntmachung des Hinweises auf die Patenterteilung: **02.12.2009**
(21) Anmeldenummer: 03702573.1
(22) Anmeldetag: 30.01.2003
(51) Int. Cl.: C08L 5/04, A61K 9/20, A23P 1/04, D01F 1/10

(54) **VORRICHTUNG UND VERFAHREN ZUR HERSTELLUNG EINER VERNETZEN SUBSTANZ, INSBESONDERE ALS MIKROKAPSEL ODER SCHICHT**
DEVICE AND METHOD FOR PRODUCING A CROSS-LINKED SUBSTANCE, ESPECIALLY IN THE FORM OF A MICROCAPSULE OR LAYER
DISPOSITIF ET PROCEDE DE FABRICATION D'UNE SUBSTANCE RETICULEE, EN PARTICULIER SOUS FORME DE MICROCAPSULE OU DE COUCHE

(30) Priorität: 30.01.2002 DE 10203629
(43) Veröffentlichungstag der Anmeldung: 27.10.2004
(73) Patentinhaber: Fraunhofer-Gesellschaft zur Förderung der angewandten Forschung e.V., 80686 München (DE)
(72) Erfinder: ZIMMERMANN, Ulrich, 97295 Waldbrunn (DE); ZIMMERMANN, Heiko, 66113 Saarbrücken (DE)
(74) Vertreter: Hertz, Oliver
(86) Internationale Anmeldenummer: PCT/EP2003/000952
(87) Internationale Veröffentlichungsnummer: WO 2003/064515

(56) Entgegenhaltungen:
- EP-A- 1 072 636
- WO-A-93/14264
- WO-A-96/03205
- US-A- 4 849 289
- TOMIDA H ET AL: "PREPARATION OF THEOPHYLLINE-LOADED CALCIUM ALGINATE GEL CAPSULES AND EVALUATION OF THEIR DRUG RELEASE CHARACTERISTICS" CHEMICAL AND PHARMACEUTICAL BULLETIN, PHARMACEUTICAL SOCIETY OF JAPAN. TOKYO, JP, Bd. 41, Nr. 12, 1. Dezember 1993 (1993-12-01), Seiten 2161-2165, XP000422467 ISSN: 0009-2363
- DATABASE WPI Section Ch, Week 198141 Derwent Publications Ltd., London, GB; Class A14, AN 1981-74623D XP002238784 & JP 56 107082 A (MITSUBISHI RAYON CO LTD) , 25. August 1981 (1981-08-25)

## Beschreibung

Die Erfindung betrifft eine Vorrichtung zur Herstellung von einer vernetzten Substanz in Form von Mikrokapseln, Formkörpern oder Schichten, insbesondere für biologische oder medizinische Anwendungen, mit einer ersten Abgabeeinrichtung zur Abgabe eines Strahls einer vernetzungsfähigen Substanz und einer Vernetzungseinrichtung zur Applizierung eines Vernetzungsmittels auf die vernetzungsfähige Substanz.

Aus Zimmermann, Ulrich et al.: Microencapsulation-Based Cell Therapy, Biotechnology Volume 10, ISBN 3-527-28320-X ist es bekannt, sogenannte Mikrokapseln zur Inkorporation von medizinischen Wirkstoffen zu verwenden. Derartige Mikrokapseln können beispielsweise aus ionotropen Gelen bestehen, die durch Applikation von Ionen vernetzen und aushärten und dadurch den zu verabreichenden medizinischen Wirkstoff umhüllen. Eine medizinische Anwendung von Mikrokapseln besteht bspw. in der Transplantationsmedizin. Mikrokapseln werden mit einem Wirkstoff (z. B. medizinisch wirksame Substanz, Insulin-produzierenden Zellen oder Gewebe) in den zu behandelnden Patienten (z. B. Leber oder intramuskulär) transplantiert. Beim genannten Beispiel würde ohne eine Umhüllung das körpereigene Immunsystem des Patienten die fremden Zellen erkennen und abstoßen. Durch die Umhüllung der Zellen mit der Mikrokapsel wird dies vorteilhaft verhindert. Die fremden, verkapselten Zellen sind wirksam vom körpereigenen Immunsystem getrennt, wohingegen die Hülle der Mikrokapsel für den darin enthaltenen oder produzierten medizinischen Wirkstoff permeabel ist. Als ionotrope Gele werden in der Transplantationsmedizin bspw. Alginate verwendet.

Der Anwendungsbereich der herkömmlichen Wirkstoffverkapselung ist beschränkt, da alle Verkapselungen mit biokompatiblen Polymeren bisher lediglich begrenzte Vernetzungsdichten und damit eine geringe Zuverlässigkeit ermöglichen. Zwar kann bei der herkömmlichen Verkapselung z. B. mit Alginaten die Permeabilität von Kapseln durch Erhöhung der Alginatkonzentration gesenkt werden. Damit ist aber auch eine erhöhte Viskosität verbunden, die die Bildung der Kapseln beschränkt.

Aus der eingangs erwähnten Veröffentlichung ist weiterhin eine Vorrichtung zur Herstellung derartiger Mikrokapseln bekannt, bei der ein Gemisch aus Alginaten und den zu verabreichenden medizinischen Wirkstoffen durch eine Düse abgegeben wird, wobei der austretende Strahl durch eine koaxiale Düsenanordnung mit Luft angeblasen wird, um die Formung kleiner Tropfen des Gemischs zu fördern. Der auf diese Weise erzeugte Tröpfchenstrahl fällt dann in ein Fällungsbad mit einem Vernetzungsmittel (z. B. eine Lösung mit Barium-, Calcium-, Eisen- oder Lanthan-Ionen) wobei die Ionen zu einer Vernetzung der Alginate und damit zu einer Kapselbildung führen.

Nachteilig an der bekannten Vorrichtung ist die Tatsache, dass die Vernetzung der Alginattröpfchen in dem Fällungsbad von der Oberfläche der Alginattröpfchen ausgeht und nach innen fortschreitet. Zuerst entsteht auf der Tröpfchenoberfläche eine vernetzte Schicht, mit der Folge, dass die divalenten Kationen nicht mehr frei in die tieferen Bereiche der Tröpfchen vordringen. Im Ergebnis kann es zu einer inhomogenen Vernetzung der Mikrokapseln kommen. Es können Instabilitäten der Mikrokapseln auftreten, die bei der späteren Anwendung, bspw. bei Auftreten von Scherkräften während der Transplantation, zu feinsten Rissen führen. Die Trennung von Zellen und Immunsystem ist dann nicht mehr gewährleistet. Es kann zu Abstoßungsreaktionen kommen.

Ein weiterer Nachteil der bekannten Vorrichtung ergibt sich aus der Beschränkung von deren Funktion auf die Herstellung vernetzter Mikrokapseln. Andere geometrische Formeln vernetzter Substanzen sind mit dem Eintropfprinzip nicht realisierbar.

Aus EP 1 072 636 A1 ist eine Vernetzungsvorrichtung bekannt, die mittels einer Düse einen Strahl einer vernetzungsfähigen Substanz abgibt, der mit Licht bestrahlt wird, was zur Vernetzung führt. Ferner sind aus WO93/14264 A1 und US-A-4 849 289 Vernetzungsverfahren aus dem Gebiet der Textiltechnik bekannt.

Der Erfindung liegt die Aufgabe zugrunde, die vorstehend beschriebene bekannte Vorrichtung zur Herstellung von Mikrokapseln zu verbessern, wobei auch eine möglichst homogene Vernetzung einer vernetzungsfähigen Substanz und ein erweiterter Anwendungsbereich angestrebt werden.

Diese Aufgaben wird durch eine Vorrichtung gemäß Patentanspruch 1 gelöst. Vorteilhafte Ausführungsformen und Anwendungen der Erfindung ergeben sich aus den abhängigen Ansprüchen.

Die Erfindung umfasst die allgemeine technische Lehre, das Vernetzungsmittel mit einem Strahl des Vernetzungsmittels zu applizieren, der auf eine zunächst unvernetzte oder eine vorvernetzte Substanz (allgemein auch: Kapselsubstanz) gerichtet wird. Die unvernetzte Substanz wird zum Beispiel als Strahl in Form von Mikrotröpfchen, eines Fadens oder einer Schicht, oder als unvernetzte Schicht der vernetzungsfähigen Substanz auf einem Träger mit dem Vernetzungsmittel versetzt. Die vorvernetzte (noch nicht vollständig vernetzte) Substanz wird zum Beispiel durch eine erste Vernetzung der vernetzungsfähigen Substanz mit dem Strahl des Vernetzungsmittels oder in einem Fällungsbad vernetzt, bevor sie einer weiteren Vernetzung mit einem weiteren Strahl des Vernetzungsmittels unterzogen wird. Die Erfindung ermöglicht allgemein die Vernetzung von Formkörper, die an die jeweilige Anwendung angepasst sind und vorzugsweise Mikrokapseln, Formkörper zum Beispiel für Implantatzwecke oder Schichten umfassen.

Vorzugsweise ist der Strahl des Vernetzungsmittels so schnell, dass das Vernetzungsmittel in die unvernetzte oder noch nicht vollständig vernetzte Substanz, z. B. Mikrokapseln eindringt. Dies bietet den Vorteil, dass der Vernetzungsprozess nicht auf die oberflächennahen Bereiche der vernetzungsfähigen Substanz, z. B. die Oberfläche von Mikrokapseln oder von Schichten auf Trägern beschränkt ist, sondern auch das Innere der Mikrokapseln oder Schichten erfasst.

Weiterhin ist vorzugsweise vorgesehen, dass der Strahl des Vernetzungsmittels so langsam ist, dass das Vernetzungsmittel nach dem Eindringen in die unvernetzte oder noch nicht vollständig vernetzte Substanz nicht wieder austritt. Dies ist vorteilhaft, da das Vernetzungsmittel nach einem Austreten aus der Substanz nicht mehr zur Vernetzung beitragen kann.

Die den beiden vorstehend genannten Randbedingungen genügende Strahlgeschwindigkeit kann in einfacher Weise durch Versuche ermittelt werden, indem für verschiedene Werte der Strahlgeschwindigkeit der erzielte Vernetzungsgrad in der vernetzungsfähigen Substanz untersucht wird. Eine verfügbare Untersuchungsmethode ist insbesondere eine konfokal-mikroskopische Abbildung des vernetzten Materials, beispielsweise zum Nachweis der Vernetzung und/oder von ausgefällten Metallresten. Die Geschwindigkeit des Strahls des Vernetzungsmittels kann dann in einfacher Weise durch eine Variation des Drucks auf den Partikelstrahl eingestellt werden, wobei Druckwerte zwischen 1 bar und 2 bar gut geeignet sind.

Vorteilhaft ist hierbei, dass das Vernetzungsmittel beim Eindringen in die zunächst unvernetzte oder noch nicht vollständig vernetzte Substanz bewirkt, dass Einschusskanäle gebildet werden, die die Vernetzung im Innern des Materials fördern. Die Einschusskanäle können insbesondere bei hochviskösen Alginaten (z. B. 40 mPa s⁻¹ oder höher) auch im vernetzten Zustand der Substanz zumindest teilweise bestehen bleiben und dadurch ggf. eine Diffusion eines Wirkstoffs durch die Einschusskanäle nach außen fördern. Damit wird die Diffusionshemmung der Kapsel herabgesetzt. Dies ist insbesondere für Anwendungen bei der Wirkstoff-Freisetzung dann wichtig, wenn größere Moleküle eingekapselt werden, wie beispielsweise Antikörper oder Faktor VIII bzw. Fragmente dieses Faktors.

Alternativ können die Vernetzungsbedingungen gerade so gewählt werden, dass die Permeabilität des vernetzten Materials bspw. für den Nährstoff- oder Sauerstoffaustausch für die Freisetzung von therapeutischen Faktoren herabgesetzt oder bspw. für die Immunisolation des verkapselten Materials sogar vollständig unterbunden wird. Beispielsweise kann die Permeabilität bei der Vernetzung von Alginaten durch die Konzentration der vernetzungsfähigen Ausgangslösung (Alginatlösung) und/oder durch einen schichtweisen Aufbau der Kapseln eingestellt werden. Bei der Vernetzung von Alginat mit Bariumpartikeln wird vorzugsweise eine Alginatkonzentration von 0.7 % (w/v) eingestellt. Bei einer geringeren Konzentration von z. B. 0.65 % (w/v) wird eine Permeabilität eingestellt, die einen Hindurchtritt von Stoffen mit einem Molekulargewicht von etwa 9000 kDA ermöglicht. Ein schichtweise Aufbau der Kapseln bedeutet, dass zum Beispiel innen mit Kalzium und außen mit Barium vernetzt wird, so dass die Permeabilität innen größer als außen ist.

Der Begriff "Wirkstoff" ist hier allgemein zu verstehen und umfasst beispielsweise einzelne Zellen, Zellgruppen oder Zellbestandteile, menschliches oder tierisches Gewebe, oder medizinisch wirksame Substanzen, wie z. B. pharmazeutische Substanzen oder Hormone.

Darüber hinaus ist in einer bevorzugten Ausführungsform der Erfindung zur Förderung der Tropfenbildung bzw. der Kapselformung als Abgabeeinrichtung eine Düsenanordnung vorgesehen, die den Strahl der Kapselsubstanz mit einem formgebenden Strahl anströmt. Vorzugsweise umgibt der formgebende Strahl hierbei den Strahl der Kapselsubstanz beispielsweise koaxial. Alternativ kann die Erfindung mit anderen an sich bekannten Abgabeeinrichtungen zur Tropfen-, Strahl- oder Schichtformung umgesetzt werden.

In einer Variante der Erfindung verläuft der Strahl des Vernetzungsmittels im wesentlichen rechtwinklig zu dem Strahl der Kapselsubstanz. Vorzugsweise verläuft der Strahl der Kapselsubstanz hierbei im wesentlichen senkrecht von oben nach unten (vertikal), wobei die Abgabeeinrichtung für den Strahl des Vernetzungsmittels seitlich neben dem Strahl der Kapselsubstanz angeordnet ist. Der Strahl der Kapselsubstanz kann jedoch auch beliebige Winkel von 0° bis 90° zur Senkrechten aufweisen.

Der Strahl des Vernetzungsmittels weist hierbei vorzugsweise einen Strahlaufweitungswinkel zwischen 10° und 170° in senkrechter und/oder waagerechter Richtung auf, wobei jedoch auch andere Werte möglich sind. Als besonders vorteilhaft hat sich ein Strahlaufweitungswinkel zwischen 10° und 30° erwiesen. Der optimale Wert der Strahlaufweitung hängt jedoch von dem verwendeten Gerät ab und sollte so gewählt werden, dass einerseits die Bewegungsenergie des abgegebenen Vernetzungsmittels durch die Strahlaufweitung möglichst wenig herabgesetzt wird und andererseits der Strahl der noch unvernetzten Mikrokapseln von dem Strahl des Vernetzungsmittels ausreichend erfasst wird.

In einer anderen Variante der Erfindung ist die Abgabeeinrichtung für den Strahl des Vernetzungsmittels dagegen ringförmig und umgibt den Strahl der Kapselsubstanz. Der Strahl des Vernetzungsmittels verläuft hierbei vorzugsweise radial von außen nach innen, wobei der Strahl des Vernetzungsmittels vorzugsweise einen vorgegebenen Anströmwinkel gegenüber dem Strahl der Kapselsubstanz aufweist.

Der Anströmwinkel des Strahls des Vernetzungsmittels gegenüber dem Strahl der Kapselsubstanz kann nahezu beliebige Werte im Bereich zwischen 0° und 90° annehmen, wobei sich Werte von mehr als 15° als besonders vorteilhaft erwiesen haben.

Bei einer ringförmigen Abgabeeinrichtung für den Strahl des Vernetzungsmittels sind vorzugsweise über den Umfang der Abgabeeinrichtung verteilt mehrere Austrittsdüsen angeordnet, so dass das Vernetzungsmittel von verschiedenen Seiten auf den Strahl der Kapselsubstanz auftrifft, wodurch eine optimale Einwirkung des Vernetzungsmittels auf die Kapselsubstanz erreicht wird.

In einer anderen Variante der Erfindung weisen die Abgabeeinrichtung für die Kapselsubstanz, die Abgabeeinrichtung für das Vernetzungsmittel und die Düsenanordnung für den formgebenden Strahl aneinander angrenzende Austrittsöffnungen auf. Beispielsweise ist es möglich, dass die Kapselsubstanz durch eine mittige Düse abgegeben wird, die von einer koaxialen Düse für den formgebenden Strahl umgeben ist, während das Vernetzungsmittel durch eine außen liegende, ebenfalls koaxiale Düse abgegeben wird. Vorzugsweise verläuft der Strahl des Vernetzungsmittels hierbei im wesentlichen koaxial zu dem Strahl der Kapselsubstanz, wobei der Strahl des Vernetzungsmittels den Strahl der Kapselsubstanz vorzugsweise umgibt.

In der bevorzugten Ausführungsform der Erfindung ist die Vernetzungseinrichtung und/oder die Abgabeeinrichtung für die Kapselsubstanz druckluftbetrieben. Beispielsweise kann das Vernetzungsmittel durch Partikelstromgeräte abgegeben werden, die aus der zahnmedizinischen Technik bekannt sind, wie beispielsweise Dentatech Prophy AP II, Dentatech Selector AP oder Kavo Prophyflex 5.

Ferner besteht die Möglichkeit, dass zur weiteren Verbesserung der Vernetzung der Mikrokapsel zusätzlich ein Fällungsbad vorgesehen ist, das ebenfalls Vernetzungsmittel enthält. Das Fällungsbad sollte als Maßnahme zur Nachvernetzung hinreichend groß sein, dass der Strahl der Kapselsubstanz tatsächlich in das Fällungsbad gelangt, da die Bestrahlung mit dem Vernetzungsmittel zu einer seitlichen Ablenkung des Vernetzungsmittels führen kann. Alternativ kann das Fällungsbad als Maßnahme zur Vorvernetzung vorgesehen sein. Vorvernetztes Material (zum Beispiel Kapseln) kann aus dem Fällungsbad entnommen, zum Beispiel auf einem festen Träger angeordnet und einer Nachvernetzung mit einem Strahl des Vernetzungsmittels unterzogen werden.

Alternative Ausführungsformen der erfindungsgemäßen Vorrichtung sind auf die Bereitstellung mindestens einer weiteren Abgabeeinrichtung und die Vernetzung von Schichten bspw. auf Trägern gerichtet. Durch die Verwendung von zwei oder mehr Abgabeeinrichtungen können vorteilhafterweise neben dem genannten Vernetzungsmittel mindestens ein weiteres Vernetzungsmittel, Wirkstoffe, Alginatpulver oder -kristalle oder biologische Substanzen in das Volumen der vernetzungsfähigen Substanz eingeschossen werden. Damit werden die Anwendungsmöglichkeiten von vernetzungsfähigen Substanzen, insbesondere vernetzungsfähigen Biopolymeren, wie z. B. Alginaten erheblich erweitert werden.

Eine bevorzugte Anwendung der Erfindung besteht in der Verkapselung biologisch oder medizinisch wirksamer (d. h. auch transformierter) Zellen in ionotropen Gelen, z. B. Alginaten. Bei dieser Anwendung wird die Geschwindigkeit des zugeführten Vernetzungsmittels so gewählt, dass die inkorporierten Zellen durch den Strahl des Vernetzungsmittels nicht verletzt werden. Eine Geschwindigkeitsgrenze wird durch Versuche insbesondere in Abhängigkeit von den verwendeten Partikeln des Vernetzungsmittels gewählt.

Darüber hinaus lässt sich die erfindungsgemäße Vorrichtung auch in biotechnologischen Prozessen einsetzen, in denen immobilisierte Zellen (z. B. Mikroorganismen, tierische Zellen, Hefezellen und pflanzliche Zellen sowie pflanzliche Protoplasten) zur Produktion und Sezernierung von Wirkstoffen, Aminosäuren, Primär-und Sekundärmetaboliten eingesetzt werden.

Weiterhin kann die Erfindung - wie bereits vorstehend genannt - zur sogenannten "Controlled Drug Release" in Organismen (insbesondere Tiere oder Menschen) verwendet werden, wobei eine therapeutisch wirksame Substanz in die Zelle eingeschlossen wird. Die Permeabilität der Mikrokapsel für die Substanz wird hierbei so eingestellt, dass die Substanz nach der Implantation der Mikrokapseln kontrolliert freigesetzt wird. Hierbei kann die therapeutisch wirksame Substanz auch in sogenannten Liposomen (Lipidvesikel) eingeschlossen und dann verkapselt werden. Vorteilhafterweise können insbesondere die folgenden Anwendungen erfindungsgemäß vernetzter Alginate bei der Wirkstofffreisetzung realisiert werden.

Zur Bekämpfung von Tumoren (z. B. im Gehirn) kann in Mikrokapseln Endostatin oder humane monoklonale Antikörper (MW 60000 kDA oder höher) eingebettet und die Permeabilität so eingestellt werden, dass die Wirkstoffe je nach den Anforderungen langsam freigesetzt werden. Die Einstellung der Permeabilität erfolgt bspw. über die Konzentration der Alginatlösung (siehe oben) und über die Wahl des Vernetzungsmittels oder der Menge des Vernetzungsmittels.

In erfindungsgemäß vernetzte Mikrokapseln können für diagnostische Zwecke, z. B. bei der NMR-Bildgebung Kontrastmittel, wie z. B. Gadoliniumderivate (insbesondere Magnevist, registrierte Marke) eingebettet werden. Vorteilhafterweise könnte damit verhindert werden, dass abweichend von einem schnellen Abbau und einer Ausscheidung über die Niere bei herkömmlichen Kontrastmitteln nunmehr eine langsame Freisetzung erzielt wird. Andere Anwendungen betreffen die Einbettung von Medikamenten, z. B. Antibiotika oder von Magnetpartikeln.

Die erfindungsgemäß bevorzugt verwendete vernetzungsfähige Substanz ist ein Alginat. Es wird vorzugsweise eine Lösung eines hochviskosen Alginats verwendet. Die Viskosität ist vorzugsweise höher als 15 mPa s. Alternativ kann Alginat mit geringerer Viskosität (bis rd. 1 bis 5 mPa s) verwendet werden, wobei in diesem Fall die Konzentration der Alginatlösung vorzugsweise auf rd. 2 bis 3 % (w/v) eingestellt wird.

Die Erfindung umfasst als weitere allgemeine technische Lehre, dass zunächst eine an sich herkömmliche Vernetzung der vernetzungsfähigen Substanz (zum Beispiel Mikrokapsel) in einem Fällungsbad vorgesehen ist, das ein Vernetzungsmittel enthält. Anschließend wird das vernetzte Material von dem Fällungsbad getrennt (zum Beispiel aus dem Fällungsbad entnommen), und auf einem festen Träger einer weiteren Vernetzung oder anderen Modifizierung mit einem Strahl von mindestens einem weiteren Vernetzungsmittel, Wirkstoffen, Pulver oder Kristallen der vernetzungsfähigen Substanz (zum Beispiel Alginatpulver oder - kristalle) oder biologischen Substanzen in das Volumen der im Fällungsbad vernetzten Substanz unterzogen.

Andere vorteilhafte Weiterbildungen der Erfindung sind in den Unteransprüchen gekennzeichnet oder werden nachstehend zusammen mit der Beschreibung der bevorzugten Ausführungsbeispiele anhand der Figuren näher erläutert. Es zeigen:
- Fig. 1:: eine erfindungsgemäße Vorrichtung zur Herstellung von biologischen Mikrokapseln aus Alginaten, wobei ein Alginatstrahl seitlich mit einem Vernetzungsmittel bestrahlt wird,
- Fig. 2:: ein alternatives Ausführungsbeispiel einer derartigen Vorrichtung, bei dem der Alginatstrahl durch eine ringförmige Düsenanordnung mit Vernetzungsmittel bestrahlt wird,
- Fig. 3:: eine Detailansicht der ringförmigen Düsenanordnung aus Fig. 2,
- Fig. 4:: ein alternatives Ausführungsbeispiel einer derartigen Vorrichtung, bei dem die Austrittsöffnungen für das Alginat, die Anblasluft und das Vernetzungsmittel unmittelbar benachbart sind,
- Fig. 5:: eine Detailansicht der Düsenanordnung der Vorrichtung aus Fig. 4,
- Fig. 6:: eine abgewandelte Ausführungsform einer erfindungsgemäßen Vorrichtung zur Herstellung von biologischen Mikrokapseln aus Alginaten, und
- Fig. 7:: weitere Ausführungsformen der erfindungsgemäßen Vorrichtung zur Herstellung von vernetzten Alginaten.

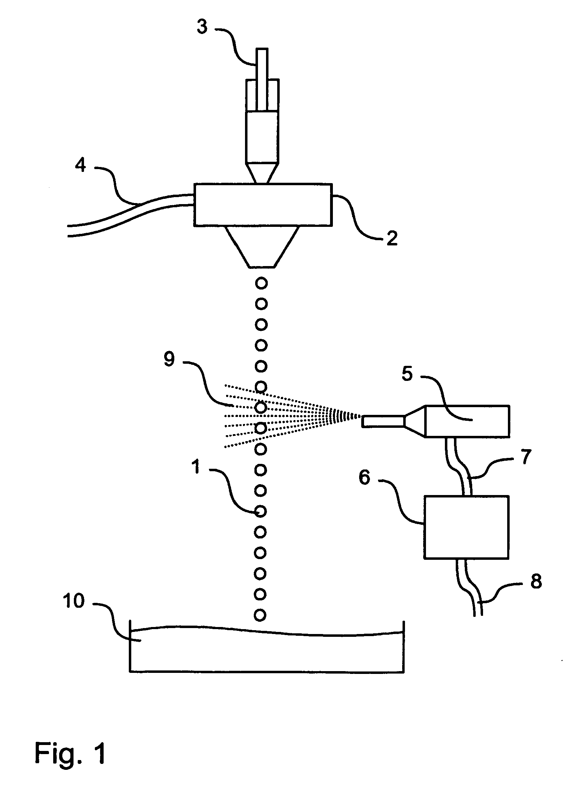

Die in Fig. 1 dargestellte erfindungsgemäße Vorrichtung dient zur Herstellung von biologischen Mikrokapseln 1 aus vernetzungsfähigen Alginaten, wobei die Mikrokapseln 1 verschiedene biologische Wirkstoffe (zum Beispiel Zellen, Gewebe, biologisch oder medizinisch wirksame Substanzen, wie Hormone) enthalten können. Die Einhüllung der biologischen Wirkstoffe in die Mikrokapseln 1 bietet den Vorteil, das diese nach einer Inkorporierung nicht von dem körpereigenen Immunsystem abgestoßen werden, da das Material der Mikrokapseln 1 bioverträglich ist.

Die erfindungsgemäße Vorrichtung weist als erste Abgabeeinrichtung eine Verkapselungsdüse 2 auf, der über einen Stempel 3 ein flüssiges Gemisch aus Alginaten und den zu verabreichenden biologischen Wirkstoffen zugeführt wird, wobei die Verkapselungsdüse 2 zusätzlich mit einer Druckluftleitung 4 verbunden ist.

Es ist jedoch auch möglich, dass das Gemisch aus Alginaten und den zu verabreichenden Wirkstoffen der Verkapselungsdüse 2 durch eine herkömmliche Einwegspritze zugeführt wird. Alternativ ist kann die erste Abgabeeinrichtung eine der Vorrichtungen zur Tropfenerzeugung unter der Wirkung elektrischer Felder sein, wie sie beispielsweise im Buch von W. M. Kühtreiber et al. (Hrsg.) "Cell encapsualtion technology and Therapeutics" (Birkhäuser, Boston) beschrieben sind.

Das Gemisch wird dann zur Formung der Mikrokapseln 1 von einem koaxialen Druckluftstrahl angeblasen, so dass die Verkapselungsdüse 2 eine strahlförmige Tropfenfolge der Mikrokapseln 1 abgibt.

Die Verkapselungsdüse 2 ist hierbei so angeordnet, dass der Strahl der Mikrokapseln 1 im wesentlichen senkrecht von oben nach unten gerichtet ist.

Seitlich neben dem Strahl der Mikrokapseln 1 ist als zweite Abgabeeinrichtung ein Partikelstrahlgerät 5 angeordnet, das von einer Basiseinheit 6 über eine Leitung 7 mit einem unter Druck stehenden Vernetzungsmittel versorgt wird.

Hinsichtlich des einsetzbaren Vernetzungsmittels wird auf die bereits eingangs erwähnte Veröffentlichung "Microencapsulation-Based Cell Therapy" sowie auf DE 199 35 231 verwiesen, so dass auf eine detaillierte Beschreibung der zahlreichen möglichen Vernetzungsmittel verzichtet werden kann.

Die Basiseinheit 6 für das Partikelstrahlgerät 5 ist über eine Druckluftleitung 8 mit einer Druckluftquelle verbunden, die zur Vereinfachung nicht dargestellt ist.

Das Partikelstrahlgerät 5 kann beispielsweise ein Dentatech Prophy AP II, ein Dentatech Selector AP oder Kavo Prophyflex 5 sein, wobei diese Geräte aus der zahnmedizinischen Technik bekannt sind.

Bei diesem Ausführungsbeispiel der erfindungsgemäßen Vorrichtung gibt das Partikelstrahlgerät 5 einen Strahl 9 des Vernetzungsmittels ab, wobei der Strahl 9 im wesentlichen waagerecht in Richtung des Strahls der Mikrokapseln 1 verläuft. Hierbei weist der Strahl 9 des Vernetzungsmittels eine Strahlaufweitung von rund 30° sowohl in senkrechter Richtung als auch in waagrechter Richtung auf, um eine ausreichende Vernetzung der Mikrokapseln 1 zu erreichen.

Die Geschwindigkeit der Partikel des Strahls 9 ist hierbei so groß, dass die einzelnen Partikel des Vernetzungsmittels in die noch nicht vernetzten Mikrokapseln 1 eindringen, so dass der Vernetzungsprozess nicht auf die oberflächennahen Bereiche der Mikrokapseln 1 beschränkt ist, sondern auch das Innere der Mikrokapseln 1 erfasst.

Die Geschwindigkeit der Partikel des Strahls 9 ist jedoch nicht so groß, dass die einzelnen Partikel des Strahls 9 nach dem Eindringen in die Mikrokapseln 1 diese wieder verlassen, da der Vernetzungsprozess dadurch beeinträchtigt würde, oder ggf. Zellen in den Mikrokapseln beschädigen.

Der Strahl 9 des Vernetzungsmittels besteht vorzugsweise aus einem diskontinuierlichen Strahl von Partikeln des Vernetzungsmittels. Die Partikel können je nach dem Arbeitsprinzip des Partikelstrahlgerätes atomare oder mikroskopische Dimensionen besitzen. Vorzugsweise werden vom Partikelstrahlgerät 5 flüssige, suspendierte oder feste Partikel mit einer charakteristischen Größe im Bereich von 500 nm bis 100 µm abgegeben. Es besteht beispielsweise die Möglichkeit, dass das Partikelstrahlgerät 5 eine flüssige Vernetzungslösung (z.B. 20 mM Ba-oder Cl-Lösung) abgibt. Besonders vorteilhaft ist es auch, wenn die Vernetzungslösung in Lipidvesikeln eingeschlossen ist, die dann in die Alginatkugeln eingeschossen werden. Bei einer Temperaturerhöhung wird die Lipidmembran dann permeabel und die divalenten Kationen werden freigesetzt.

Schließlich ist unterhalb der Verkapselungsdüse 2 noch ein herkömmliches Fällungsbad 10 angeordnet, das ebenfalls Vernetzungsmittel enthalten und dadurch zu einer Verbesserung der Vernetzung der Mikrokapseln 1 führen kann. Derartige Fällungsbäder sind aus den beiden vorstehend erwähnten Veröffentlichungen bekannt, so dass hier auf eine detaillierte Beschreibung von Fällungsbäder verzichtet werden kann.

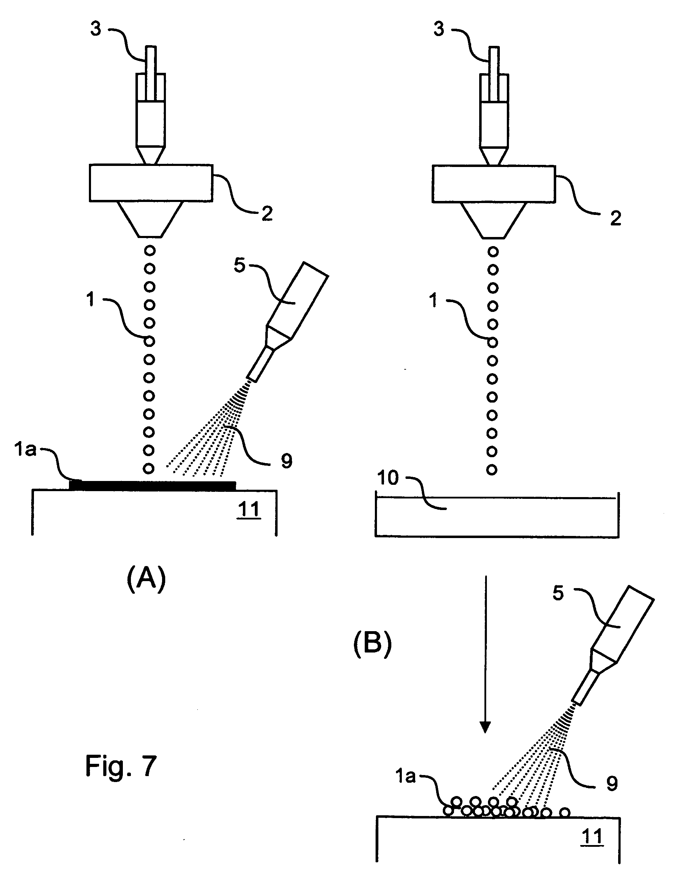

Die Formgestaltung und/oder die Betriebsweise der Verkapselungsdüse 2 ist gemäß Figur 1 zum Beispiel so gewählt, dass der Strahl der vernetzungsfähigen Substanz (Alginat) aus einer Vielzahl von Mikrotropfen besteht. Die Erfindung ist nicht auf die Vernetzung von Mikrotropfen beschränkt. Vielmehr kann die Verkapselungsdüse 2 so betrieben oder gestaltet werden, dass andere Strahlformen erzeugt werden. Beispielsweise kann bei verändertem Betriebsdruck die Unterteilung des Strahls in Mikrotropfen aufgehoben und stattdessen ein kontinuierlicher Alginatfaden erzeugt werden. Der Alginatfaden kann eine Querschnittsform je nach der Gestalt der Verkapselungsdüse 2 besitzen. Beispielsweise kann der Alginatfaden mit einem kreisförmigen Querschnitt gebildet sein (Durchmesser z. B. im Bereich von 50 µm bis 5 mm oder größer). Diese Gestaltung ermöglicht die Erzeugung zylindrischer Alginatkörper, wie sie bspw. als Implantatkörper von Interesse sind. Alternativ kann die Düse die Form eines Schlitzes besitzen. In diesem Fall bildet der Strahl der Alginatlösung einen schichtförmigen Flüssigkeitsvorhang, der analog zu den oben erläuterten Prinzipien vernetzt wird. Je nach Form der Verkapselungsdüse können andere, zum Beispiel gekrümmte Schichtformen erzeugt werden. Die Bildung von vernetzten Alginatschichten auf Substraten wird unten unter Bezug auf Fig. 7 erläutert.

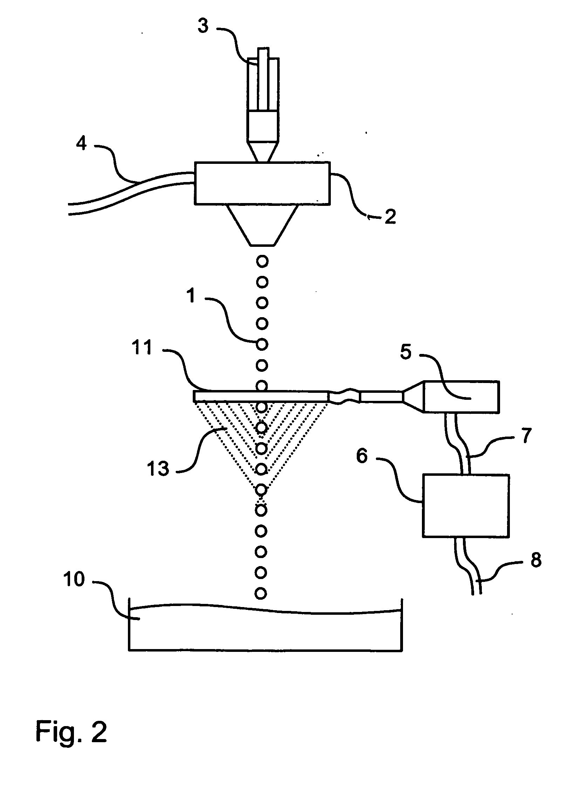

Das in Fig. 2 dargestellte Ausführungsbeispiel einer erfindungsgemäßen Vorrichtung stimmt weitgehend mit dem vorstehend beschriebenen und in Fig. 1 dargestellten Ausführungsbeispiel überein, so dass im Folgenden für übereinstimmende Bauteile dieselben Bezugszeichen verwendet werden und zur Vermeidung von Wiederholungen im wesentlichen auf die vorstehende Beschreibung zu Fig. 1 verwiesen wird.

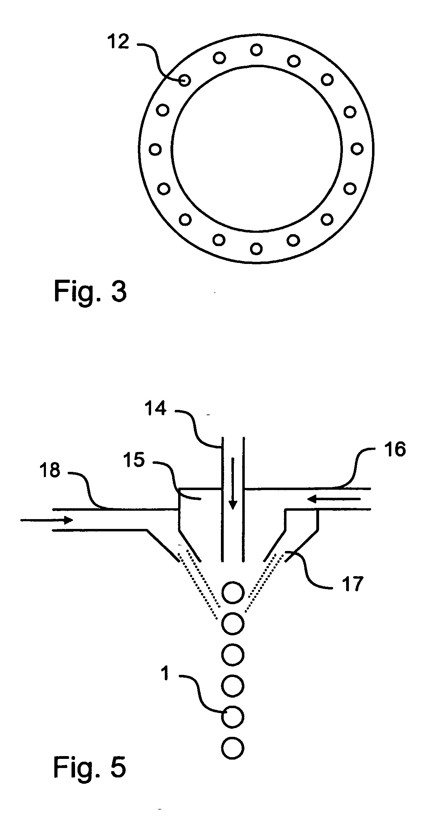

Die Besonderheit dieses Ausführungsbeispiels besteht im Wesentlichen darin, dass das Partikelstrahlgerät 5 mit einer ringförmigen Düsenanordnung 11 verbunden ist, die detailliert in Fig. 3 dargestellt ist.

Die Düsenanordnung 11 umgibt den Strahl der Mikrokapseln 1 ringförmig und weist über ihren Umfang verteilt zahlreiche Düsenöffnungen 12 auf, durch die ein fächerförmiger Strahl 13 des Vernetzungsmittels abgegeben wird. Der Strahl 13 des Vernetzungsmittels weist hierbei gegenüber dem Strahl der Mikrokapseln 1 einen Anströmwinkel von rund 40° auf und verläuft ansonsten radial nach innen, so dass der Strahl 13 des Vernetzungsmittels auf die Mikrokapseln 1 auftrifft.

Auch hierbei ist die Geschwindigkeit des Strahls 13 so groß, dass das Vernetzungsmittel in die einzelnen Mikrokapseln 1 eindringt, jedoch so langsam, dass das Vernetzungsmittel anschließend nicht wieder austritt.

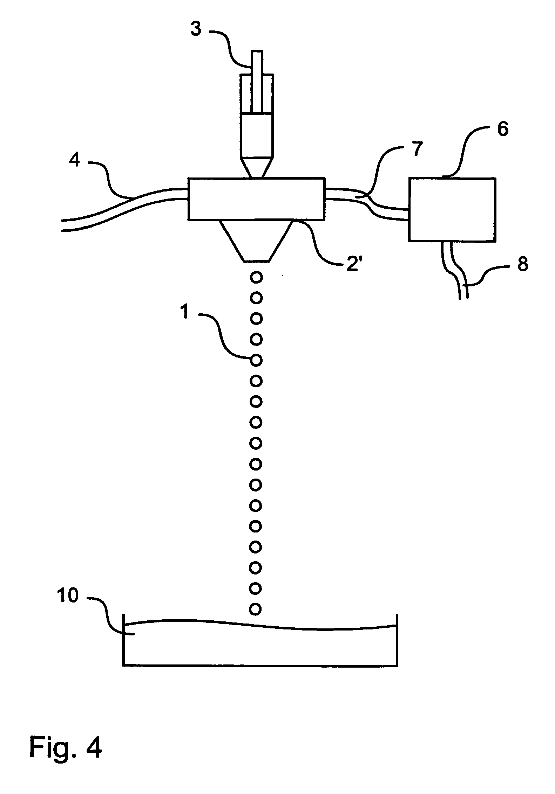

Schließlich stimmt auch das in Fig. 4 dargestellte Ausführungsbeispiel einer erfindungsgemäßen Vorrichtung weitgehend mit den vorstehend beschriebenen und in Fig. 1 dargestellten Ausführungsbeispiel überein, so dass auch im Folgenden für einander entsprechende Bauteile dieselben Bezugszeichen verwendet werden und zur Vermeidung von Wiederholungen auf die vorstehende Beschreibung verwiesen wird.

Die Besonderheit dieses Ausführungsbeispiels besteht in der konstruktiven Ausgestaltung einer Verkapselungsdüse 2', die in Fig. 5 detailliert dargestellt ist.

So weist die Verkapselungsdüse 2' eine zentrische Düse 14 zur Abgabe des Alginat-Zell-Gemischs auf, wobei die Düse 14 von einer konzentrischen Düse 15 umgeben ist, die über eine Zuleitung 16 mit Druckluft versorgt wird. Die aus der Düse 15 austretende Druckluft fördert hierbei die Formung feiner Tröpfchen aus dem Alginat-Zell-Gemisch, das aus der Düse 14 austritt. Die Düse 15 kann entsprechend zur Bildung anderer Strahlformen gestaltet und/oder betrieben werden.

Die Düse 15 ist schließlich von einer ebenfalls ringförmigen Düse 17 umgeben, die über eine Zuleitung 18 mit dem Vernetzungsmittel versorgt wird. Das Vernetzungsmittel tritt also aus der Düse 17 aus, wobei der austretende Strahl des Vernetzungsmittels neben der axialen Geschwindigkeitskomponente auch eine Radialkomponente aufweist und deshalb auf die einzelnen Mikrokapseln 1 auftrifft, was zu deren Vernetzung führt.

Die Geschwindigkeit des aus der Düse 17 austretenden Strahls des Vernetzungsmittels ist hierbei wieder so groß, dass das Vernetzungsmittel in die einzelnen Mikrokapseln 1 eindringt, ohne diese anschließend wieder zu verlassen.

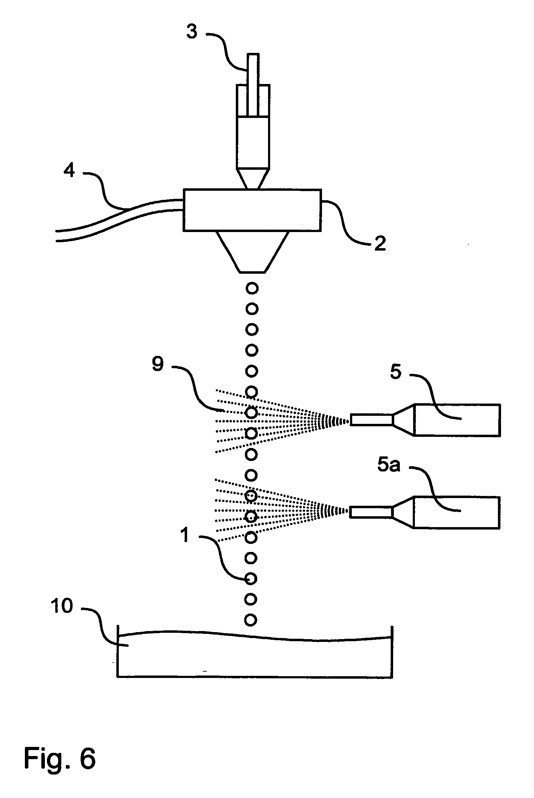

Figur 6 illustriert eine abgewandelte Ausführungsform der Erfindung, bei der zusätzlich zu der oben beschriebenen Abgabeeinrichtung 5 zur Abgabe des Vernetzungsmittels mindestens eine weitere Abgabeeinrichtung 5a entlang der Fallstrecke der Alginattropfen angeordnet ist. Die Abgabeeinrichtung 5a ist vorzugsweise wie die oben beschriebene Abgabeeinrichtung 5 aufgebaut. Durch die Verwendung von mehreren Abgabeeinrichtungen können die folgenden neuen Anwendungen bei der Herstellung von kapsel- oder schichtförmigem Alginatmaterial realisiert werden.

Die verschiedenen Abgabeeinrichtungen 5, 5a können verschiedene Vernetzungsmittel in das Alginat mit verschiedenen Geschwindigkeiten einschießen, so dass verschiedene Bereiche im Alginat mit verschiedenen Ionen vernetzt werden. Beispielsweise wird über die erste Abgabeeinrichtung 5 Calciumchlorid mit einer hohen Geschwindigkeit abgegeben, so dass im Kern der Kapseln 1 eine Ca-Vernetzung erfolgt, während in der zweiten Abgabeeinrichtung 5a Bariumchloridkristalle abgegeben werden. Die Geschwindigkeit der Bariumchloridkristalle wird so eingestellt, dass sie nur die äußeren Schichten des Alginats erreichen. Die Herstellung von innen Ca- und außen Ba-vernetzten Mikrokapseln mit eingeschlossenen biologischen Materialien (z. B. Zellen, Gewebe) besitzt den Vorteil, dass in der Umgebung der biologischen Materialien das wesentlich physiologischer wirksame Ca vorhanden ist, während in den oberflächennahen Bereichen Ba vorherrscht, welches eine höhere Stabilität der Vernetzung ermöglicht. Ein weiterer Vorteil besteht in der Beeinflussung der Permeabilität der Mikrokapseln. Im Inneren der Mikrokapsel ist die Diffusion größer als in der Peripherie, die mit Ba vernetzt ist.

Des Weiteren kann die zusätzliche Abgabeeinrichtung 5a zur Injektion von biologischen Faktoren vorgesehen sein. Es können bspw. mesenchymalen Stammzellen eingeschlossen sein, wobei zunächst eine Ba-Vernetzung und anschließend eine Injektion von Wachstumshormonen in fester Form erfolgt. Alternativ können an der zusätzlichen Abgabeeinrichtung 5a therapeutische Faktoren (Wirkstoffe) in die vernetzten Alginate eingeschossen werden.

Eine weitere Anwendung der zusätzlichen Abgabeeinrichtung 5a besteht in der Zufuhr von Alginatpulver- oder kristallen. Bei dieser Gestaltung kann die lokale Alginatkonzentration in der Mikrokapsel oder dem anderweitig geformten Alginatkörper variiert werden. Es können Alginat-Zusammensetzungen hergestellt werden, die aus einem lokal definierten Mischungsverhältnis von unterschiedlichen Alginaten bestehen. Derartige Alginatzusammensetzungen bilden Verbundwerkstoffe mit neuen physikalischen und chemischen Eigenschaften. Diese äußern sich insbesondere in neuen Freisetzungsgenetiken von therapeutischen Faktoren aus Zellen und Gewebe oder bei der "controlled drug release".

Schließlich ist möglich, mit der zusätzlichen Abgabeeinrichtung 5a andere Hydrogele oder auch hydrophobe Polymermikropartikel einzuschießen. Im letzteren Fall könnten innerhalb des vernetzten Materials Mikrodomänen als Reservoir für hydrophobe (lipidlösliche) Faktoren geschaffen werden. Es könnten ferner thermotrope Hydrogele (z. B. Agarose) eingeschossen werden. Dies besitzt den Vorteil, dass die Freisetzungsgenetik der Mikrokapsel durch eine lokale Erwärmung (Mikrowellentherapie) beschleunigt werden kann. Schließlich kann eine Wirkstofffreisetzung auch nach innen erfolgen, um bspw. verkapselte Zellen im Zeitverlauf biochemisch zu triggern.

Es können auch Zucker eingeschossen werden, die die Wasserstruktur und damit die Permeabilität der Kapseln bei verschiedenen Temperaturen verändern. Bei Anwendungen in der Lebensmitteltechnologie können bspw. Farbstoffe zugeführt werden, um die Alginate zu färben.

Figur 7A, B illustriert weitere Abwandlungen der Erfindung, bei denen die Kapselsubstanz im unvernetzten oder einem vernetzten Zustand auf einem Träger einer weiteren Vernetzung und/oder Modifizierung unterzogen werden. Gemäß Figur 7A treffen die Mikrotropfen 1 zum Beispiel aus gelöstem Alginat auf die Oberfläche eines Trägers 11, auf dem die Alginatlösung eine Schicht 1a bildet. Die Abgabeeinrichtung 5 ist in diesem Fall auf die Schicht 1a gerichtet, um vernetzte, trägergebundene Substanzen herzustellen. Abweichend von der Illustration kann die Oberfläche des Trägers 11 gekrümmt gebildet sein. Die vernetzte Alginatschicht ist entsprechend mit der Krümmung gebildet.

Die Gestaltung gemäß Figur 7A kann insbesondere im Rahmen der Nanotechnologie verwendet werden. Die Vernetzung der vernetzungsfähigen Substanz erfolgt zur Bildung eines Schutzmantels und/oder eines Carriersystems für technische Geräte mit charakteristischen Dimensionen im µm- bis nm-Bereich. Es können auch Wirkstoffe als Verbrauchsstoffe in nanotechnologische Geräte eingeschossen werden (Einschuss von Energievorräten).

Gemäß Figur 7B werden die Mikrotropfen zunächst in einem Fällungsbad vernetzt, wie es an sich bekannt ist. Anschließend werden die vernetzten Kapseln 1a schichtförmig auf einem Träger 11 angeordnet und dort entsprechend den oben erläuterten Prinzipien mit der Abgabeeinrichtung 5 einer weiteren Vernetzung und/oder Modifizierung unterzogen. Die Trennung der vernetzten Kapseln 1a vom Fällungsbad kann zum Beispiel durch Abfiltern der vernetzten Kapseln 1a oder ein Ablassen des Fällungsbades erfolgen. Es kann zusätzlich eine Vorvernetzung analog zu Figur 1 vorgesehen sein.

Die Erfindung ist nicht auf die vorstehend beschriebenen bevorzugten Ausführungsbeispiele beschränkt. Vielmehr ist eine Vielzahl von Varianten, Kombinationen und Abwandlungen möglich, die von dem Erfindungsgedanken Gebrauch machen und deshalb ebenfalls in den Schutzbereich fallen.

## Patentansprüche

1. Vorrichtung zur Herstellung einer vernetzten Substanz in Form von Mikrokapseln (1) mit darin eingebetteten Medikamenten, Formkörpern für Implantatzwecke oder Alginatschichten aus einer vernetzungsfähigen Kapselsubstanz, insbesondere aus vernetzungsfähigen Alginaten, die umfasst:
a) eine erste Abgabeeinrichtung (2) zur Abgabe der vernetzungsfähigen Kapselsubstanz, und
b) eine Vernetzungseinrichtung zur Applizierung eines Vernetzungsmittels auf die Kapselsubstanz,
**dadurch gekennzeichnet,**
c) **dass** die Vernetzungseinrichtung mindestens eine zweite Abgabeeinrichtung (5) aufweist, die einen Strahl (9, 13) des Vernetzungsmittels auf die Kapselsubstanz richtet, und
d) **dass** der Strahl (9) des Vernetzungsmittels Partikel des Vernetzungsmittels in flüssiger, suspendierter oder fester Form enthält.

2. Vorrichtung nach Anspruch 1, **dadurch gekennzeichnet, dass** die erste Abgabeeinrichtung (2) die Kapselsubstanz in Form eines Strahls abgibt.

3. Vorrichtung nach Anspruch 2, **dadurch gekennzeichnet, dass** die zweite Abgabeeinrichtung (5) den Strahl (9, 13) des Vernetzungsmittels auf den Strahl oder eine Schicht der Kapselsubstanz richtet.

4. Vorrichtung nach Anspruch 3, **dadurch gekennzeichnet, dass** die Vernetzungseinrichtung mindestens eine dritte Abgabeeinrichtung (5a) aufweist, die einen Strahl eines Vernetzungsmittels, einer Wirksubstanz oder eines Zusatzstoffs auf den Strahl oder die Schicht der Kapselsubstanz richtet.

5. Vorrichtung nach mindestens einem der vorhergehenden Ansprüche, **dadurch gekennzeichnet, dass** der Strahl (9, 13) des Vernetzungsmittels so schnell ist, dass das Vernetzungsmittel in die unvernetzte Kapselsubstanz eindringt.

6. Vorrichtung nach Anspruch 5, **dadurch gekennzeichnet, dass** der Strahl (9, 13) des Vernetzungsmittels so langsam ist, dass das Vernetzungsmittel nach dem Eindringen in die unvernetzten Kapselsubstanz nicht wieder austritt.

7. Vorrichtung nach mindestens einem der Ansprüche 2 bis 6, **dadurch gekennzeichnet, dass** zur Strahlformung eine Verkapselungsdüse (2) und/oder eine Düsenanordnung (15) vorgesehen ist, die den Strahl der Kapselsubstanz mit einem formgebenden Strahl anströmt, wobei der formgebende Strahl den Strahl der Kapselsubstanz umgibt.

8. Vorrichtung nach Anspruch 7, **dadurch gekennzeichnet, dass** die Verkapselungsdüse (2) und/oder die Düsenanordnung (15) zur Bildung eines in Mikrotropfen unterteilten Strahls oder eines kontinuierlichen Strahls eingerichtet ist.

9. Vorrichtung nach Anspruch 7, **dadurch gekennzeichnet, dass** die Verkapselungsdüse (2) und/oder die Düsenanordnung (15) dazu eingerichtet ist, den kontinuierlichen Strahl mit einem vorbestimmten Durchmesser oder in Schichtform zu bilden.

10. Vorrichtung nach mindestens einem der Ansprüche 2 bis 9, **dadurch gekennzeichnet, dass** der Strahl (9, 13) des Vernetzungsmittels in einem Winkel zwischen 15° und 90° zu dem Strahl der Kapselsubstanz verläuft.

11. Vorrichtung nach mindestens einem der Ansprüche 2 bis 10, **dadurch gekennzeichnet, dass** die mindestens eine zweite Abgabeeinrichtung (11) ringförmig ist und den Strahl der Kapselsubstanz umgibt.

12. Vorrichtung nach Anspruch 11, **dadurch gekennzeichnet, dass** der Strahl (9, 13) des Vernetzungsmittels gegenüber dem Strahl der Kapselsubstanz einen vorgegebenen Anströmwinkel aufweist und ansonsten radial nach innen verläuft.

13. Vorrichtung nach Anspruch 11 oder 12, **dadurch gekennzeichnet, dass** die mindestens eine zweite Abgabeeinrichtung (11) mehrere Austrittsdüsen aufweist, die über den Umfang der zweiten Abgabeeinrichtung (11) verteilt angeordnet sind.

14. Vorrichtung nach mindestens einem der vorhergehenden Ansprüche, **dadurch gekennzeichnet, dass** die erste Abgabeeinrichtung (2') für die Kapselsubstanz und/oder die zweite Abgabeeinrichtung (17, 18) für das Vernetzungsmittel und/oder die Düsenanordnung (15) für den formgebenden Strahl aneinander angrenzende Austrittsöffnungen aufweisen.

15. Vorrichtung nach Anspruch 14, **dadurch gekennzeichnet, dass** der Strahl (9, 13) des Vernetzungsmittels im wesentlichen koaxial zu dem Strahl der Kapselsubstanz verläuft.

16. Vorrichtung nach Anspruch 15, **dadurch gekennzeichnet, dass** der Strahl (9, 13) des Vernetzungsmittels den Strahl der Kapselsubstanz umgibt.

17. Vorrichtung nach mindestens einem der vorhergehenden Ansprüche, **dadurch gekennzeichnet, dass** die Vernetzungseinrichtung (5) und/oder die erste Abgabeeinrichtung (2, 2') für die Kapselsubstanz druckluftbetrieben ist.

18. Vorrichtung nach mindestens einem der vorhergehenden Ansprüche, **dadurch gekennzeichnet, dass** die mindestens eine zweite Abgabeeinrichtung ein zahnmedizinisches Partikelstrahlgerät (5) ist.

19. Vorrichtung nach mindestens einem der Ansprüche 2 bis 18, **dadurch gekennzeichnet, dass** der Strahl der Kapselsubstanz zwischen der Austrittsöffnung der ersten Abgabeeinrichtung und dem Auftreffpunkt des Strahls (9) des Vernetzungsmittels eine freie Länge zwischen 5 cm und 80 cm aufweist.

20. Vorrichtung nach mindestens einem der Ansprüche 2 bis 19, **dadurch gekennzeichnet, dass** der Strahl der Kapselsubstanz im wesentlichen senkrecht von oben nach unten verläuft, wobei an der Unterseite ein Auffangbecken (10) angeordnet ist.

21. Vorrichtung nach Anspruch 20, **dadurch gekennzeichnet, dass** das Auffangbecken (10) ein Vernetzungsmittel enthält.

22. Vorrichtung nach Anspruch 21, **dadurch gekennzeichnet, dass** das Auffangbecken (10) die Vernetzungseinrichtung darstellt, ein Träger (11) zur Aufnahme der im Auffangbecken (10) vernetzten Kapselsubstanz vorgesehen ist und die mindestens eine zweite Abgabeeinrichtung (5) dazu vorgesehen ist, einen Strahl (9) des Vernetzungsmittels oder einer Zusatzsubstanz auf die Kapselsubstanz auf dem Träger (11) zu richten.

23. Vorrichtung nach mindestens einem der vorhergehenden Ansprüche 1 bis 19, **dadurch gekennzeichnet, dass** der Strahl der Kapselsubstanz im wesentlichen senkrecht von oben nach unten verläuft, wobei an der Unterseite ein Träger (11) angeordnet ist, auf dessen Oberfläche die mindestens eine Abgabeeinrichtung (5) der Vernetzungseinrichtung gerichtet ist.

24. Vorrichtung nach mindestens einem der vorhergehenden Ansprüche, **dadurch gekennzeichnet, dass** die Kapselsubstanz Alginat enthält.

25. Vorrichtung nach mindestens einem der vorhergehenden Ansprüche, **dadurch gekennzeichnet, dass** das Vernetzungsmittel freie oder gelöste Ionen oder Salzkristalle enthält.

## Claims

1. A device for producing a cross-linked substance in the form of microcapsules
(1) having medicaments embedded therein, moulds for implantation purposes or alginate layers from a cross-linkable capsule substance, in particular from cross-linkable alginates, comprising
a) a first dispensing apparatus (2) for dispensing the cross-linkable capsule substance, and
b) a cross-linking apparatus for applying a cross-linking agent on the capsule substance
**characterized in that**
c) the cross-linking apparatus has at least a second dispensing apparatus (5) directing a jet (9, 13) of the cross-linking agent onto the capsule substance, and
d) the jet (9) of the cross-linking agent contains particles of the cross-linking agent in liquid, suspended or solid form.

2. The device according to claim 1, **characterized in that** the first dispensing apparatus (2) dispenses the capsule substance in the form of a jet.

3. The device according to claim 2, **characterized in that** the second dispensing apparatus (5) directs the jet (9, 13) of the cross-linking agent onto the jet or a layer of the capsule substance.

4. The device according to claim 3, **characterized in that** the cross-linking apparatus has at least a third dispensing apparatus (5a) directing a jet of a cross-linking agent, an active substance or an additive onto the jet or the layer of the capsule substance.

5. The device according to at least one of the preceding claims, **characterized in that** the jet (9, 13) of the cross-linking agent is so fast that the cross-linking agent penetrates into the non-linked capsule substance.

6. The device according claim 5, **characterized in that** the jet (9, 13) of the cross-linking agent is so slow that the cross-linking agent does not re-emerge after penetrating into the non-linked capsule substance.

7. The device according to at least one of the claims 2 to 6, **characterized in that** for shaping the jet an encapsulation nozzle (2) and/or a nozzle arrangement (15) are provided which stream against the jet of the capsule substance with a shaping jet, the shaping jet surrounding the jet of the capsule substance.

8. The device according to claim 7, **characterized in that** the encapsulation nozzle (2) and/or the nozzle arrangement (15) are arranged to form a jet divided into micro droplets or a continuous jet.

9. The device according to claim 7, **characterized in that** the encapsulation nozzle (2) and/or the nozzle arrangement (15) are arranged to form the continuous jet with a predetermined diameter or in the form of a layer.

10. The device according to at least one of the claims 2 to 9, **characterized in that** the jet (9, 13) of the cross-linking agent flows in an angle between 15° and 90° related to the jet of the capsule substance.

11. The device according to at least one of the claims 2 to 10, **characterized in that** the at least one second dispensing apparatus (11) is of annular shape and surrounds the jet of the capsule substance.

12. The device according to claim 11, **characterized in that** the jet (9, 13) of the cross-linking agent has a predetermined streaming angle related to the jet of the capsule substance and otherwise flows radially inwards.

13. The device according to claim 11 or 12, **characterized in that** the at least one second dispensing apparatus (11) has a plurality of outlet nozzles which are in a distributed arrangement over the periphery of second dispensing apparatus (11).

14. The device according to at least one of the preceding claims, **characterized in that** the first dispensing apparatus (2') for the capsule substance and/or the second dispensing apparatus (17, 18) for the cross-linking agent and/or the nozzle arrangement (15) for the shaping jet have adjacent outlet apertures.

15. The device according to claim 14, **characterized in that** the jet (9, 13) of the cross-linking agent substantially flows coaxially related to the jet of the capsule substance.

16. The device according to claim 15, **characterized in that** the jet (9, 13) of the cross-linking agent surrounds the jet of the capsule substance.

17. The device according to at least one of the preceding claims, **characterized in that** the cross-linking apparatus (5) and/or the first dispensing apparatus (2, 2') for the capsule substance are air-pressure operated.

18. The device according to at least one of the preceding claims, **characterized in that** the at least one second dispensing apparatus is a dental particle jet instrument (5).

19. The device according to at least one of the claims 2 to 18, **characterized in that** the jet of the capsule substance between the outlet aperture of the first dispensing apparatus and the impact point of the jet (9) of the cross-linking agent has a free length between 5 cm and 80 cm.

20. The device according to at least one of the claims 2 to 19, **characterized in that** the jet of the capsule substance substantially flows vertically top-down, a collecting basin (10) being arranged at the bottom side.

21. The device according to claim 20, **characterized in that** the collecting basin (10) contains a cross-linking agent.

22. The device according to claim 21, **characterized in that** the collecting basin (10) represents the cross-linking apparatus, a carrier (11) is provided for receiving the capsule substance cross-linked in the collecting basin (10) and the at least one second dispensing apparatus (5) is provided to direct a jet (9) of the cross-linking agent or an additive onto the capsule substance on the carrier (11).

23. The device according to at least one of the claims 1 to 19, **characterized in that** the jet of the capsule substance substantially flows vertically top-down, a carrier (11) being arranged at the bottom side, onto the surface of which the at least one dispensing apparatus (5) of the cross-linking apparatus is directed.

24. The device according to at least one of the preceding claims, **characterized in that** the capsule substance contains alginate.

25. The device according to at least one of the preceding claims, **characterized in that** the cross-linking agent contains free or dissolved ions or salt crystals.

## Revendications

1. Dispositif de fabrication d'une substance réticulée sous forme de microcapsules (1) dans lesquelles sont encapsulés des médicaments, de corps profilés destinés à des implantations ou de couches d'alginate, à partir d'une substance d'encapsulage réticulable, en particulier à partir d'alginates réticulables, lequel dispositif comporte :
a) un premier dispositif de distribution (2) destiné à distribuer la substance d'encapsulage réticulable, et
b) un dispositif de réticulation destiné à appliquer un agent réticulant sur la substance d'encapsulage,
**caractérisé**
c) **en ce que** le dispositif de réticulation comporte au moins un deuxième dispositif de distribution (5), qui projette un jet (9, 13) d'agent réticulant sur la substance d'encapsulage, et
d) **en ce que** le jet (9) d'agent réticulant contient des particules d'agent réticulant sous forme liquide, en suspension ou solide.

2. Dispositif selon la revendication 1, **caractérisé en ce que** le premier dispositif de distribution (2) distribue la substance d'encapsulage sous la forme d'un jet.

3. Dispositif selon la revendication 2, **caractérisé en ce que** le deuxième dispositif de distribution (5) projette le jet (9, 13) d'agent réticulant sur le jet de substance d'encapsulage ou une couche de substance d'encapsulage.

4. Dispositif selon la revendication 3, **caractérisé en ce que** le dispositif de réticulation comporte au moins un troisième dispositif de distribution (5a), qui projette un jet d'un agent réticulant, d'une substance active ou d'un adjuvant sur le jet de substance d'encapsulage ou la couche de substance d'encapsulage.

5. Dispositif selon au moins l'une des revendications précédentes, **caractérisé en ce que** le jet (9, 13) d'agent réticulant est si rapide que l'agent réticulant pénètre dans la substance d'encapsulage non réticulée.

6. Dispositif selon la revendication 5, **caractérisé en ce que** le jet (9, 13) d'agent réticulant est si lent que l'agent réticulant, après avoir pénétré dans la substance d'encapsulage non réticulée, n'en ressort pas.

7. Dispositif selon au moins l'une des revendications 2 à 6, **caractérisé en ce que** pour la formation d'un jet, il est prévu une buse (2) et/ou un système de buses (15), qui projettent sur le jet de substance d'encapsulage un jet de formage, ledit jet de formage entourant le jet de substance d'encapsulage.

8. Dispositif selon la revendication 7, **caractérisé en ce que** la buse (2) et/ou le système de buses (15) sont conçus pour former un jet divisé en gouttes microscopiques ou un jet continu.

9. Dispositif selon la revendication 7, **caractérisé en ce que** la buse (2) et/ou le système de buses (15) sont conçus pour former le jet continu avec un diamètre prédéterminé ou en forme de nappe.

10. Dispositif selon au moins l'une des revendications 2 à 9, **caractérisé en ce que** le jet (9, 13) d'agent réticulant forme avec le jet de la substance d'encapsulage un angle entre 15° et 90°.

11. Dispositif selon au moins l'une des revendications 2 à 10, **caractérisé en ce que** ledit au moins un deuxième dispositif de distribution (11) est réalisé avec une forme annulaire et entoure le jet de substance d'encapsulage.

12. Dispositif selon la revendication 11, **caractérisé en ce que** le jet (9, 13) d'agent réticulant forme un angle d'afflux prédéfini par rapport au jet de substance d'encapsulage et, pour le reste, s'étend radialement vers l'intérieur.

13. Dispositif selon la revendication 11 ou 12,
**caractérisé en ce que** ledit au moins un deuxième dispositif de distribution (11) comporte plusieurs buses de sortie, qui sont réparties sur le pourtour du deuxième dispositif de distribution (11).

14. Dispositif selon au moins l'une des revendications précédentes, **caractérisé en ce que** le premier dispositif de distribution (2') de substance d'encapsulage et/ou le deuxième dispositif de distribution (17, 18) d'agent réticulant et/ou le système de buses (15) pour le jet de formage comportent des orifices de sortie adjacents les uns aux autres.

15. Dispositif selon la revendication 14, **caractérisé en ce que** le jet (9, 13) d'agent réticulant est sensiblement coaxial au jet de substance d'encapsulage.

16. Dispositif selon la revendication 15, **caractérisé en ce que** le jet (9, 13) d'agent réticulant entoure le jet de substance d'encapsulage.

17. Dispositif selon au moins l'une des revendications précédentes, **caractérisé en ce que** le dispositif de réticulation (5) et/ou le premier dispositif de distribution (2, 2') de la substance d'encapsulage sont actionnés par air comprimé.

18. Dispositif selon au moins l'une des revendications précédentes, **caractérisé en ce que** ledit au moins un deuxième dispositif de distribution est un projecteur de particules (5) pour la dentisterie.

19. Dispositif selon au moins l'une des revendications 2 à 18, **caractérisé en ce que** le jet de substance d'encapsulage entre l'orifice de sortie du premier dispositif de distribution et le point d'impact du jet (9) d'agent réticulant a une longueur libre entre 5 cm et 80 cm.

20. Dispositif selon au moins l'une des revendications 2 à 19, **caractérisé en ce que** le jet de substance d'encapsulage est orienté sensiblement perpendiculairement du haut vers le bas, une cuve de réception (10) étant disposée sur le côté inférieur.

21. Dispositif selon la revendication 20, **caractérisé en ce que** la cuve de réception (10) contient un agent réticulant.

22. Dispositif selon la revendication 21, **caractérisé en ce que** la cuve de réception (10) constitue le dispositif de réticulation, il est prévu un support (11) pour recevoir la substance d'encapsulage réticulée dans la cuve de réception (10), et ledit au moins un deuxième dispositif de distribution (5) est prévu pour diriger un jet (9) d'agent réticulant ou d'un additif sur la substance d'encapsulage sur le support (11).

23. Dispositif selon au moins l'une des revendications 1 à 19, **caractérisé en ce que** le jet de substance d'encapsulage est orienté sensiblement perpendiculairement du haut vers le bas, sachant que sur le côté inférieur est disposé un support (11), sur la surface duquel est dirigé ledit au moins un dispositif de distribution (5) du dispositif de réticulation.

24. Dispositif selon au moins l'une des revendications précédentes, **caractérisé en ce que** la substance d'encapsulage contient un alginate.

25. Dispositif selon au moins l'une des revendications précédentes, **caractérisé en ce que** l'agent réticulant contient des ions libres ou dissous ou des cristaux de sel.
